(19) Europäisches Patentamt
European Patent Office
Office européen des brevets

(11) Veröffentlichungsnummer: **0 036 120**
**B1**

(12) # EUROPÄISCHE PATENTSCHRIFT

(45) Veröffentlichungstag der Patentschrift: **09.02.83**

(51) Int. Cl.³: **C 07 C 149/42,** C 07 C 148/00

(21) Anmeldenummer: **81101477.8**

(22) Anmeldetag: **02.03.81**

(54) **Verfahren zur Herstellung von Bis-(amino-phenyl)-disulfiden.**

(30) Priorität: **14.03.80 DE 3009846**

(43) Veröffentlichungstag der Anmeldung: **23.09.81 Patentblatt 81/38**

(45) Bekanntmachung des Hinweises auf die Patenterteilung: **09.02.83 Patentblatt 83/6**

(84) Benannte Vertragsstaaten: **BE DE FR GB**

(56) Entgegenhaltungen: **US-A-2 435 508**

(73) Patentinhaber: **BAYER AG, Zentralbereich Patente, Marken und Lizenzen, D-5090 Leverkusen 1, Bayerwerk (DE)**

(72) Erfinder: **Blank, Heinz Ulrich, Dr., Am Geusfelde 35, D-5068 Odenthal (DE)**
Erfinder: **Pfister, Theodor, Dr., Sillerstrasse 51, D-5600 Wuppertal (DE)**
Erfinder: **Pütter, Rolf, Dr., Poststrasse 7, D-4000 Düsseldorf (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von Bis-(amino-phenyl)-disulfiden

Die Erfindung betrifft ein Verfahren zur Herstellung von Bis-(amino-phenyl)-disulfiden.

Es ist bekannt, daß man Bis-(amino-phenyl)-disulfide erhält, wenn man 2-Nitro- oder 4-Nitro-chlorbenzol zunächst mit Natriumsulfid oder Natriumsulfid/Schwefel in Wasser umsetzt und dann mit Luftsauerstoff oder Wasserstoffperoxid oxidiert (US-PS 1 933 217; DE-OS 2 053 715). Es sind auch Varianten dieses Verfahrens bekannt geworden, bei denen ohne die genannten Oxidationsmittel gearbeitet wird (DE-OS 2 503 164; DE-OS 2 758 786; US-PS 3 150 186). Die nach diesen Herstellungsmethoden erhaltenen Produkte enthalten jedoch mehr oder weniger große Mengen an Verunreinigungen, so daß für verschiedene Verwendungszwecke verlustreiche Reinigungsprozesse erforderlich werden.

Weiter ist bekannt, daß man Bis-(2-amino-phenyl)-disulfid erhält, wenn man 2-Amino-thiophenol oder sein Natriumsalz mit Dimethylsulfoxid oder mit Wasserstoffperoxid umsetzt (US-PS 3 981 809; US-PS 4 136 118). Dimethylsulfoxid und Wasserstoffperoxid sind jedoch, verglichen mit Luftsauerstoff und Schwefel, teure Oxidationsmittel.

Ebenso ist die Verwendung von Schwefel zur Herstellung von aliphatischen Disulfiden aus Mercaptanen bekannt, wobei in Gegenwart von Basen wie Alkalihydroxiden oder tertiären aliphatischen Aminen, und in Gegenwart von Alkoholen als Lösungsmittel (US-PS 3 340 324), N-Methylpyrrolidon oder Isopropanol, gegebenenfalls im Gemisch mit Wasser, als Lösungsmittel (US-PS 3 994 979) oder in aliphatischen Disulfiden als Lösungsmittel (DE-AS 1 214 220) gearbeitet wird.

Es wurde nun ein Verfahren zur Herstellung von Bis-(aminophenyl)-disulfiden der Formel

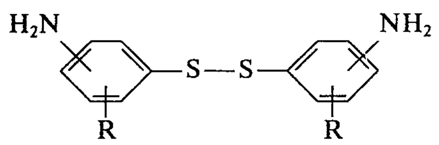

(I)

gefunden, in welcher

R für Wasserstoff oder Halogen steht,

das dadurch gekennzeichnet ist, daß man Amino-thiophenole der Formel

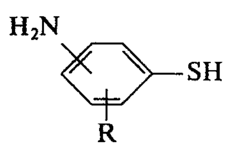

(II)

in welcher

R die oben angegebene Bedeutung hat,

mit Schwefel oder einer schwefelabgebenden Substanz in wäßriger Dispersion bei einer Temperatur von 10 bis 120° C und einem pH-Wert von 3 bis 9 umsetzt.

Als Halogen sei beispielsweise Fluor, Chlor, Brom, Jod, bevorzugt Chlor oder Brom, besonders bevorzugt Chlor, genannt.

Das erfindungsgemäße Verfahren kann am Beispiel der Umsetzung von 2-Amino-thiophenol und Schwefel durch die folgende Formelgleichung wiedergegeben werden;

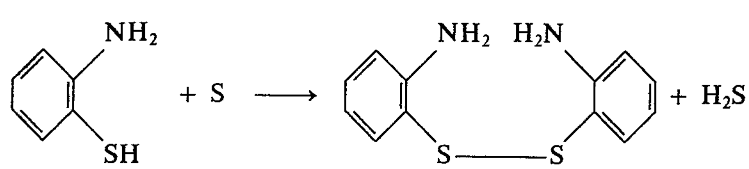

Als Amino-thiophenol für das erfindungsgemäße Verfahren seien beispielsweise genannt:

2-Amino-thiophenol, 4-Amino-thiophenol, 2-Chlor-4-amino-thiophenol, 2-Brom-4-amino-thiophenol, 4-Chlor-2-amino-thiophenol, 4-Brom-2-amino-thiophenol, 3-Chlor-2-amino-thiophenol, 6-Chlor-2-amino-thiophenol und 3-Chlor-4-amino-thiophenol.

Bevorzugte Amino-thiophenole für das erfindungsgemäße Verfahren sind solche in denen R für Wasserstoff oder Chlor steht.

Besonders bevorzugt für das erfindungsgemäße Verfahren ist der Einsatz von 2-Amino-thiophenol.

Amino-thiophenole sind bekannte Verbindungen und können nach bekannten Verfahren, beispielsweise durch Umsetzung von Chlor-nitro-benzolen mit Hydrogensulfiden oder Sulfiden hergestellt werden (DE-OS 2 127 898).

Die Amino-thiophenole werden im erfindungsgemäßen Verfahren mit Schwefel umgesetzt. Der Schwefel kann hierbei in Form von elementaren Schwefel, wie Schwefelpulver, Schwefelschuppen, Schwefelblume, oder über eine schwefelabgebende Substanz im erfindungsgemäßen Verfahren eingesetzt werden. Als schwefelabgebende Substanzen seien Polysulfide, wie Disulfide oder Trisulfide, und Thiosulfate, wie Natriumthiosulfat oder die beim Ansäuern freigesetzte Thioschwefelsäure (Gmelins Handbuch der anorganischen Chemie, 8. Auflage, Band 9, Seite 874 Verlag Chemie 1960) genannt.

Schwefel oder die schwefelabgebende Substanz werden zweckmäßigerweise im erfindungsgemäßen Verfahren in einer solchen Menge eingesetzt, daß pro Mol Amino-thiophenol 0,4 bis 0,7 Mol, vorzugsweise 0,5 bis 0,55 Mol, Schwefel vorhanden ist.

Das erfindungsgemäße Verfahren wird bei einer Temperatur von 10 bis 120°C, bevorzugt 50 bis 110°C, durchgeführt. Besonders bevorzugt wird bei Rückflußtemperatur des Reaktionsgemisches gearbeitet.

Das erfindungsgemäße Verfahren wird in der Regel bei einem Druck zwischen 0,01 und 1 bar, bevorzugt 0,5 und 1 bar durchgeführt. Besonders bevorzugt wird bei Normaldruck gearbeitet. Der pH-Wert für die Durchführung des erfindungsgemäßen Verfahrens kann im Bereich von 3 bis 9, vorzugsweise im Bereich von 4 bis 8 liegen. Bei der Verwendung von salzartigen schwefelabgebenden Substanzen, wie Natriumthiosulfat, und/oder beim Einsatz der Aminothiophenole in Form ihrer Salze wird der pH-Wert durch Zugabe einer kleinen Menge einer Mineralsäure oder organischen Säure eingestellt. Bei der Umsetzung von Amino-thiophenolen mit Schwefel stellt sich im allgemeinen der erforderliche pH-Wert ohne weitere Hilfsstoffe ein. Für den Fall, daß zur pH-Einstellung eine Säure verwandt wird, sei beispielsweise eine starke Mineralsäure, wie Salzsäure oder Schwefelsäure, eine schwache Mineralsäure, wie z. B. Kohlensäure (Kohlendioxid in Wasser) oder eine organische Säure, wie Essigsäure oder Ameisensäure, genannt.

Zur Durchführung des erfindungsgemäßen Verfahrens werden die Ausgangskomponenten bei Raumtemperatur in Wasser dispergiert und dann unter Rühren auf die Reaktionstemperatur erhitzt, wobei der bei der Umsetzung gebildete Schwefelwasserstoff entweicht. Der Schwefelwasserstoff wird nach üblichen Methoden isoliert oder umgewandelt, beispielsweise durch Absorption in Alkalihydroxidlösungen und Umwandlung in das zugehörige Alkalisalz oder durch Oxidation zu Schwefel. Gegen Ende der Umsetzung wird zur weitgehenden Entfernung des restlichen Schwefelwasserstoffs ein schwaches Vakuum an das Reaktionsgemisch gelegt oder ein Trägergasstrom, beispielsweise Stickstoff oder Luft, durch die Reaktionsmischung geleitet. Nach der Abkühlung des Reaktionsgemisches, beispielsweise auf eine Temperatur zwischen 0 und 20°C, kann das Produkt in üblicher Weise, beispielsweise durch Filtrieren, Absaugen oder Abschleudern isoliert werden.

Überraschenderweise werden die Bis-(amino-phenyl)-disulfide nach dem erfindungsgemäßen Verfahren in praktisch quantitativen Ausbeuten und in hoher Reinheit erhalten, während nach den Verfahren des Standes der Technik stets mehr oder weniger stark verunreinigte Produkte anfallen. Die nach dem erfindungsgemäßen Verfahren herstellbaren Bis-(amino-phenyl)-disulfide können als Fungizide, Beizmittel für Epoxidharze, Komponenten für Schmieröl-Additive, Komponenten für Polyurethan-Präpolymere, Kautschukpeptisierungsmittel und als Zwischenprodukte für die Synthese von Benzthiazolderivaten verwendet werden (DE-OS 2 503 164).

## Beispiel 1

25 g (0,2 Mol) 2-Amino-thiophenol und 3,5 g (0,11 Mol) Schwefel werden in 150 ml Wasser dispergiert und die Mischung wird 90 Minuten lang unter Rückfluß zum Sieden erhitzt und gerührt. Nach Abkühlen auf 20°C wird abgesaugt und das Produkt bei 40°C im Wasserstrahlvakuum getrocknet. Ausbeute 25 g (100% der theoretischen Ausbeute); Schmelzpunkt 93°C. Nach dünnschichtchromatographischer Kontrolle (Methylenchlorid an Kieselgel) ist das Produkt einheitlich Bis-(2-amino-phenyl)-disulfid.

## Beispiel 2

25 g (0,2 Mol) 2-Amino-thiophenol und 25 g (0,1 Mol) Natriumthiosulfat-pentahydrat werden in 150 ml Wasser vorgelegt und die Mischung wird 2 Stunden lang unter Rückfluß erhitzt und gerührt. Nach Abkühlen auf 20°C wird abgesaugt und das Produkt bei 40°C im Wasserstrahlvakuum getrocknet.

Ausbeute: 20,5 g (82% der theoretischen Ausbeute) Bis-(2-amino-phenyl)-disulfid; Schmelzpunkt 93° C.

## Beispiel 3

Der Versuch wird wie in Beispiel 2 beschrieben durchgeführt mit der Abweichung, daß bei ca. 80° C durch Zutropfen von Essigsäure der pH-Wert auf 5 eingestellt wird.

Ausbeute: 24,5 g (98% der Theorie) Bis-(2-amino-phenyl)-disulfid; Schmelzpunkt 93° C.

## Beispiel 4

Der Versuch wird wie im Beispiel 2 beschrieben durchgeführt mit der Abweichung, daß bei ca. 80° C Kohlendioxid eingeleitet wird und durch Zutropfen von 40%iger Schwefelsäure der pH-Wert auf 5 eingestellt wird.

Ausbeute: 24,5 g (98% der Theorie) Bis-(2-amino-phenyl)-disulfid; Schmelzpunkt 93° C.

## Patentansprüche

1. Verfahren zur Herstellung von Bis-(amino-phenyl)-disulfiden der Formel

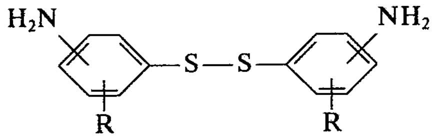

in der

R für Wasserstoff oder Halogen steht,

dadurch gekennzeichnet, daß man Amino-thiophenole der Formel

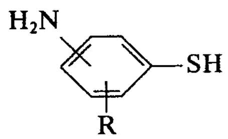

in der

R die oben angegebene Bedeutung hat,

mit Schwefel oder einer schwefelabgebenden Substanz in wäßriger Dispersion bei einer Temperatur von 10 bis 120° C und einem pH-Wert von 3 bis 9 umsetzt.

2. Verfahren nach Anspruch 1, dadurch gekennzeichnet, daß Schwefel oder eine schwefelabgebende Substanz in einer solchen Menge eingesetzt wird, daß pro Mol Amino-thiophenol 0,4 bis 0,7 Mol Schwefel zur Verfügung steht.

3. Verfahren nach Anspruch 1 und 2, dadurch gekennzeichnet, daß Schwefel oder eine schwefelabgebende Substanz in einer Menge von 0,5 bis 0,55 Mol po Mol Amino-thiophenol zur Verfügung steht.

4. Verfahren nach Anspruch 1 bis 3, dadurch gekennzeichnet, daß die Umsetzung bei 50 bis 110° C durchgeführt wird.

5. Verfahren nach Anspruch 1 bis 4, dadurch gekennzeichnet, daß die Umsetzung bei der Rückflußtemperatur der Reaktionsmischung durchgeführt wird.

6. Verfahren nach Anspruch 1 bis 5, dadurch gekennzeichnet, daß bei einem pH-Wert von 4 bis 8 gearbeitet wird.

7. Verfahren nach Anspruch 1 bis 6, dadurch gekennzeichnet, daß die Umsetzung mit elementarem Schwefel durchgeführt wird.

8. Verfahren nach Anspruch 1 bis 6, dadurch gekennzeichnet, daß die Umsetzung mit Natriumthiosulfat durchgeführt wird.

9. Verfahren nach Anspruch 1 bis 8, dadurch gekennzeichnet, daß chlor- oder bromsubstituierte Amino-thiophenole umgesetzt werden.

10. Verfahren nach Anspruch 1 bis 8, dadurch gekennzeichnet, daß nicht substituierte Amino-thiophenole umgesetzt werden.

**Claims**

1. Process for the preparation of bis-(amino-phenyl)-disulphides of the formula

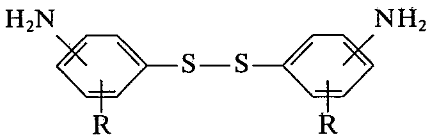

in which

R represents hydrogen or halogen,

characterised in that amino-thiophenols of the formula

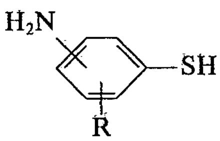

in which

R has the abovementioned meaning,

are reacted with sulphur or a sulphur-donating substance in aqueous dispersion at a temperature of 10 to 120° C and a pH value of 3 to 9.

2. Process according to Claim 1, characterised in that sulphur or a sulphur-donating substance is employed in such an amount that 0.4 to 0.7 mol of sulphur is available per mol of amino-thiophenol.

3. Process according to Claim 1 and 2, characterised in that sulphur or a sulphur-donating substance is available in an amount of 0.5 to 0.55 mol per mol of amino-thiophenol.

4. Process according to Claim 1 to 3, characterised in that the reaction is carried out at 50 to 110° C.

5. Process according to Claim 1 to 4, characterised in that the reaction is carried out at the reflux temperature of the reaction mixture.

6. Process according to Claim 1 to 5, characterised in that it is carried out at a pH value of 4 to 8.

7. Process according to Claim 1 to 6, characterised in that the reaction is carried out with elementary sulphur.

8. Process according to Claim 1 to 6, characterised in that the reaction is carried out with sodium thiosulphate.

9. Process according to Claim 1 to 8, characterised in that chlorine-substituted or bromine-substituted aminothiophenols are reacted.

10. Process according to Claim 1 to 8, characterised in that unsubstituted amino-thiophenols are reacted.

**Revendications**

1. Procédé de préparation de bis-(aminophényl)-disulfures de formule:

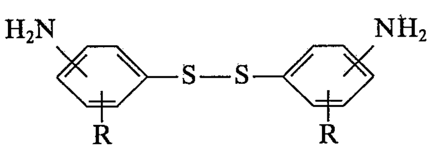

dans laquelle

R représente un atome d'hydrogène ou un atome d'halogène,

caractérisé en ce qu'on fait réagir des amino-thiophénols de formule:

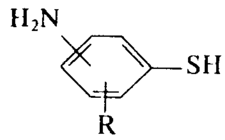

dans laquelle

R a la signification indiquée ci-dessus,

avec du soufre ou une substance donnant du soufre en dispersion aqueuse, à une température de 10 à 120°C et à un pH de 3 à 9.

2. Procédé suivant la revendication 1, caractérisé en ce qu'on utilise du soufre ou une substance donnant du soufre en une quantité calculée de telle sorte que l'on dispose de 0,4 à 0,7 mole de soufre par mole d'amino-thiophénol.

3. Procédé suivant les revendications 1 et 2, caractérisé en ce que du soufre ou une substance donnant du soufre est disponible en une quantité de 0,5 à 0,55 mole par mole d'amino-thiophénol.

4. Procédé suivant les revendications 1 à 3, caractérisé en ce qu'on effectue la réaction à une température de 50 à 110°C.

5. Procédé suivant les revendications 1 à 4, caractérisé en ce qu'on effectue la réaction à la température de reflux du mélange réactionnel.

6. Procédé suivant les revendications 1 à 5, caractérisé en ce qu'on travaille à un pH de 4 à 8.

7. Procédé suivant les revendications 1 à 6, caractérisé en ce qu'on effectue la réaction avec du soufre élémentaire.

8. Procédé suivant les revendications 1 à 6, caractérisé en ce qu'on effectue la réaction avec du thiosulfate de sodium.

9. Procédé suivant les revendications 1 à 8, caractérisé en ce qu'on fait réagir des amino-thiophénols substitués par le chlore ou le brome.

10. Procédé suivant les revendications 1 à 8, caractérisé en ce qu'on fait réagir des aminothiophénols non substitués.